# EUROPEAN PATENT SPECIFICATION

(11) **EP 2 662 073 B1**
(45) Date of publication and mention of the grant of the patent: **08.11.2017**
(21) Application number: 13167187.7
(22) Date of filing: 09.05.2013
(51) Int. Cl.: A61K 9/00, A61M 5/315, A61K 31/00, A61K 38/08, A61K 38/31

(54) **Octreotide Injection**
Octreotid-Injektion
Injection d'octréotide

(30) Priority: 09.05.2012 IN 1422MU2012
(43) Date of publication of application: 13.11.2013
(73) Proprietor: Sun Pharmaceutical Industries LTD, Andheri (East) Mumbai 400 093 (IN)
(72) Inventor: Pawar, Shantaram, 390 020 Baroda (IN); George, Alex, 390 020 Baroda (IN); Kane, Prashant, 390 020 Baroda (IN); Bhowmick, Subhas, 390 020 Baroda (IN)
(74) Representative: HGF Limited

(56) References cited:
- EP-A1- 0 496 141
- US-A1- 2008 131 513

## Description

### FIELD OF INVENTION

The present invention relates to a sterile solution of octreotide acetate present as a reservoir in multiple dose pen injection device adapted to administer a portion of the reservoir in multiple, daily doses.

### BACKGROUND OF INVENTION

Octreotide acetate, known chemically as L-cysteinamide, D-phenylalanyl-L-cysteinyl-L-phenylalanyl-D-tryptophyl-L-lysyl-L-threonyl-N-[2-hydroxy-1-(hydroxymethyl)propyl]-, cyclic (2#7)-disulfide; [R-(R*, R*)] acetate salt, is a long-acting octapeptide with pharmacological actions mimicking those of the natural hormone somatostatin. The FDA approved parenteral product is available under brand name of Sandostatin® which is a clear aqueous preserved solution filled in sterile 5 ml multidose vials in two strengths, 200 µgs/ml and1000 µgs /ml. It is indicated in the treatment of Acromegaly, Carcinoid Tumors and VIPomas. The dosage and frequency of daily administration is varied depending upon the indication, but the subcutaneous administration is necessarily done at least once daily. For instance, the dosage may be initiated at 50 µgs three times a day. The dosage most commonly found to be effective is 100 µgs three times a day, but some patients require up to 500 µgs three times a day for maximum effectiveness. In cases of Carcinoid Tumors, the suggested daily dosage of octreotide acetate during the first 2 weeks of therapy ranges from 100-600 µgs/day in 2 to 4 divided doses (mean daily dosage is 300 µgs). In case of VIPomas, daily dosages of 200to 300 µgs in 2-4 divided doses are recommended during the initial 2 weeks of therapy (range 150-750 µgs) to control symptoms of the disease. Being a daily administration therapy at least once or more than once a day, the syringe and needle form of the injection delivery system may be problematic in terms of patient compliance apart from the typical errors associated in drawing the solution from syringes and aversion about use of needle and syringe. Further, patients suffering from illness may not be capable of accurately controlling the operation of the convention syringes. Hence, it is apparent that there lies a need for a multiple dose pen injection device which can deliver accurately variable doses of octreotide acetate solution. Such a pen injection device obviates problems associated with conventional syringe and needle injection system which requires withdrawal of the solution from the vial.

The present invention precisely, takes care of these problems associated with the octreotide subcutaneous injection system of the prior art. The multiple dose pen injection device of the present invention allows self administration with precision and accuracy in withdrawing volumes which would be otherwise a concern in case of the 'syringe-vial' type of product available under the brand name of Sandostatin® Further, the multiple dose pen injector delivery device of the present invention is tailored to suit the variable octreotide dosage regimens. Due to flexibility of volume adjustments, the multiple dose pen injection device of the present invention can cater the needs of octreotide for different conditions such as Acromegaly, Carcinoid Tumors and VIPomas wherein different doses are prescribed.

### SUMMARY OF THE INVENTION

The present invention provides a sterile solution comprising octreotide acetate in a pharmaceutically acceptable vehicle, wherein solution is present as a reservoir in a multiple dose pen injection device, the device being adapted to subcutaneously inject a portion of the said reservoir in multiple, daily doses and further being adapted to provide multiple portions of the said solution, while the reservoir remains sterile. The solution has a concentration of the octreotide acetate from 2 to 20 mg/mL and comprises a preservative.

### BRIEF DESCRIPTION OF FIGURES

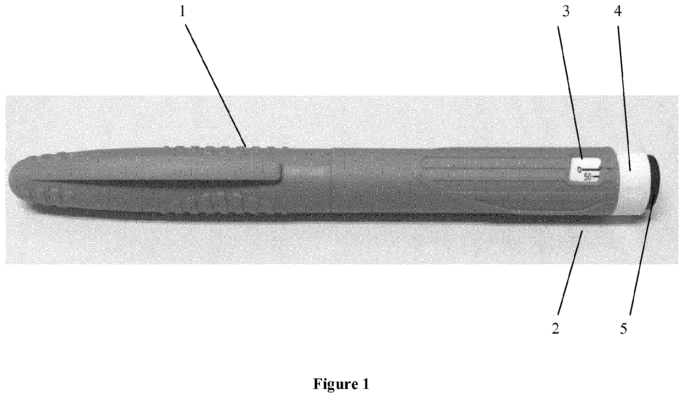

Figure 1: It represents the multiple dose pen injection device according to one embodiment of the present invention. The figure depicts a cap (1) that covers a pre-filled medicament cartridge and a dose dialing facility (2). The dose dialing facility have a dose dial with varied markings (3) along with a dose set knob (4) that allows the user to select the desired dose and a dose release button (5) which is pressed to inject the selected dose. In figure 1, two markings visible in dose dial includes '0' and '50' wherein '0' depicts the initial starting position and '50' stands for 50µg octreotide acetate.

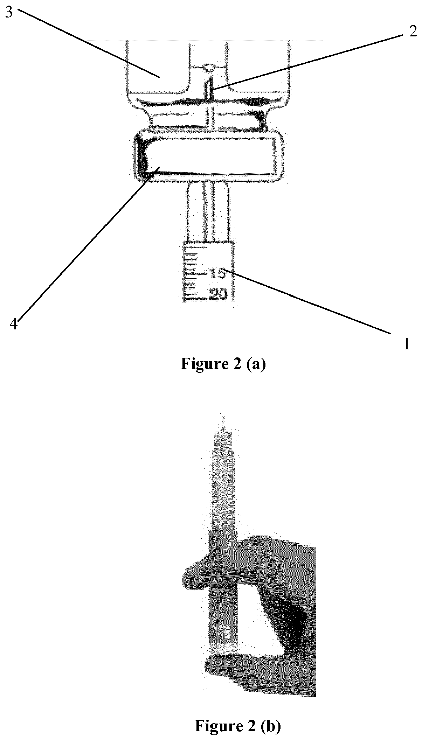

Figure 2(a): It represents the prior art 'conventional syringe-vial assembly' used to administer the marketed product - Sandostatin®. The figure depicts a conventional syringe (1) having a plunger and a needle (2), the needle being inserted in a vial (3) through a rubber stopper (4). The figure represents the process of withdrawal of the medicament liquid from the vial by use of a conventional syringe. It is evident that the approved product available in the market (Sandostatin®) makes use of a conventional syringe-vial assembly wherein administration of medicament involves manual withdrawal of the drug solution from a vial with the help of a conventional syringe followed by administration to the patient using the syringe. Such delivery method is associated with several drawbacks such as patient non-compliance, possibility of errors in withdrawing solution from syringes and aversion about use of needle and syringe. The pack insert of approved product (Sandostatin®) infact suggest how critical it is to use the product. For instance, the pack insert direct the user that when the drug level gets low, special care needs to be taken to hold the bottle straight up and down and to keep the needle tip in liquid while pulling back on the plunger. In an attempt to try to get every last drop out of the bottle, there is an increased possibility of drawing air into the syringe and getting an incorrect/incomplete dose.
Figure 2(b): It represents the multiple dose pen injection device according to one embodiment of the present invention depicting the process of priming of the pen injection device prior to first use, wherein the priming is conveniently done by adjusting the dose selection knob to marking '200' in dose dial followed by pushing the release button until a stream of medication oozes out from the needle to ensure the removal of any air bubble inside the cartridge. It is to be noted that the priming can be done simply by adjusting the dose selection knob to marking '200' in dose dial followed by pushing the release button until a stream of medication oozes out from the needle to ensure the removal of any air bubble inside the cartridge.

### DETAILED DESCRIPTION OF THE INVENTION

The present invention provides a sterile solution comprising octreotide acetate in a pharmaceutically acceptable vehicle, according to claim 1, wherein the solution is present as a reservoir in a multiple dose pen injection device, the device being adapted to subcutaneously inject a portion of the said reservoir in multiple, daily doses and further being adapted to provide multiple portions of the said solution, while the reservoir remains sterile.

The term 'multiple dose pen injection device' as used herein means an injection assembly which can house a reservoir of a multiple, sterile, doses of octreotide acetate in solution. Preferably the reservoir is in a cartridge. Further the multiple dose pen injection device includes a dose dialing facility that allows the user to select and inject a desired dose of octreotide acetate. The device includes an injection assembly in which the reservoir of octreotide acetate is in a cartridge which is in-built in the multiple dose pen injection device. The device does not involve the withdrawal of the drug solution from a separate storage container as is the case in conventional syringe-vial assembly. That is, the sterile solution of octreotide acetate can be directly administered from the pen device, such that there is no requirement of withdrawal of the octreotide solution from a separate storage container as is the case in a conventional syringe-vial assembly. The 'multiple dose pen injection device' does not include any mechanics to create a force or suction such as use of a plunger, in conventional syringe.

The sterile solution of the present invention comprises octreotide acetate which acts as a reservoir providing multiple subcutaneous doses in the form of portions of the solution. It may be important to note that the inactive ingredients or excipients in the solution of the present invention are in dissolved or soluble state and there is no particulate matter. Particularly, the solution does not include any material that may exhibit a depot or sustained release effect on the release of octreotide acetate upon injection or as such. These materials include, but are not limited to, polymers which may be synthetic or biodegradable, gelling or non-gelling, ion exchange resins, implantable material which form implant or in situ or otherwise. The sterile solution of the present invention is essentially suitable for multiple, daily administrations for consecutive days for the period of therapy as may be prescribed such as few months, for instance six to twelve months.

According to the present invention, the sterile solution comprises octreotide acetate in amount ranging from 2 mg to 20 mg per milliliter, preferably from 5 mg to 10 mg per milliliter of the solution. In one specific embodiment, the octreotide acetate is present at a concentration of 5 mg per ml. The sterile solution is suitably administered in a dose having a volume from 10 µL to 40 µL. In another aspect, the present disclosure provides a 'method of administering' octreotide acetate to a subject in need thereof, said method comprising subcutaneous injection of the solution of octreotide acetate, wherein the solution is administered directly from the multiple dose pen injection device. The multiple dose pen injection device of the present invention imparts flexibility in terms of selecting & administering varied doses of the medicament as well as the convenience of self administration. This may be of particular importance in case of administration in children, where individualization is necessary and the dose is based on body weight. The present invention, thus provides an accurate and convenient method of administration of octreotide acetate which is not available in the art. In one aspect the sterile solution of octreotide acetate is at a concentration of 5 mg/ml filled to a capacity of 3.0 ml in the cartridge of the multiple dose pen injection device of the present invention. For treatment of carcinoid tumour, solution is administered at the prescribed total daily dose of 100 µgs to 600 µgs two to four times a day. Similarly, for acromegaly therapy, 50µg of octreotide acetate is administered twice or thricedaily. In one embodiment, one multiple dose pen injection device can provide multiple portions of the divided doses of 600 µgs each to a patient in need of which can be used for a period of 25 days. For treatment of acromegaly, therapy is initiated at 50 µgs three times a day which may be titrated up to 100 µg three times a day. When the treatment of acromegaly includes total daily dose of 300 µgs in divided doses, the reservoir of the sterile solution of 3.0 ml volume having octreotide acetate at 5 mg/ml concentration can supply doses of 300 µgs each for about a period of 50 days.

The pharmaceutically acceptable vehicle of the sterile solution of the present invention may comprise one or more buffers or isotonicity agents, preservatives. In one aspect the pH of the sterile solution was adjusted to a pH in the range of 3.9 to 4.5. The pH may be adjusted with the help of pH adjusting agents such as sodium bicarbonate, sodium hydroxide or hydrochloric acid. However, it is possible to use a buffer system to maintain the pH range and isotonicity agents. Suitable buffers that may be used include, but are not limited to acetate buffer, lactate buffer, citrate buffer, gluconate buffer, tartrate buffer, phosphate buffer and the like. Typical isotonicity agents that can be used in the sterile solution include, but are not limited to, sodium chloride, potassium chloride, sorbitol, mannitol, lactose, sucrose, maltose, trehalose, dextrose and mixtures thereof. Typically, a solution having an osmolarity in the range of 300-400 mOsm/Kg gives a subcutaneous injection which is painless. According to one aspect of the present invention, when the concentration of octreotide acetate was increased five as compared to the prior art subcutaneous solution, the osmolarity of the solution was found to be in the range of 300-400 mOsm/Kg, inspite of increase in the concentration of the active ingredient in amounts as high as 5 mg per ml. The sterile solution may optionally comprise a chelating agent such as disodium-EDTA and the like.

The sterile solution is essentially a multidose preparation, that is, the same sterile solution that can be used for multiple injections. Since the multiple dose pen injection device of the present invention is for delivering multiple doses, integrity of the seal of the multiple dose pen injection device is compromised during the multiple uses thus making the solution repeatedly susceptible to exposure to non-sterile environment. The solution is thus required to include at least one suitable preservative apart from other components such as buffers or isotonicity agents. Examples of the suitable preservative include, but are not limited to, phenol, benzyl alcohol, m-cresol, methyl parabens, propylparaben, butylparaben, chlorbutanol, thimerosal, phenylmercuric salts, etc. In one preferred embodiment, phenol is used as a preservative in amount of 4 mg per mililitre to 5 mg per mililitre of the solution. In one specific aspect the solution comprises 1 mg per milliliter of octreotide acetate. The amount of phenol used is 4 mg to 5 mg per milliliter. In another embodiment, the solution comprises 5 mg per mililitre of octreotide acetate and 4 mg to 5 mg per mililitre of phenol. The present disclosure provides a method of maintaining the sterility of a reservoir of solution stored in a multiple dose pen injection device which provides multiple subcutaneous injections to a subject in need thereof. The maintenance of the sterility of the solution in the multiple dose injection device, particularly throughout the shelf life is determined by methods known such as the preservative efficacy test (PET), in which the performance of the preservative is checked. Such tests are described in literatures, such as for eg. United States Pharmacopoeia described in USP 34. The preservative maintained its potency and it was observed that it is non reactive with the components of the container or closure system. In certain embodiments, octreotide acetate is present at higher concentration for instance 5mg/ ml as compared to Sandostatin®. It was found that 4 to 5 mg per ml of phenol was sufficient to maintain the sterility of the solution, throughout the shelf life, while the pen is being used. This provides an advantage, in that a considerably reduced amount of phenol is injected per dose as compared to Sandostatin® product. This is achieved because a volume of injection is reduced since concentration of octreotide acetate in increased, for instance, five times. , The solution is subjected to "in-use stability and sterility testing". An in-use shelf-life is intended to provide assurance of the appropriate quality of the product throughout its use such that the product remains physically and chemically stable and sterile during the in-use period, thereby ensuring the safety and efficacy of the product. The European Union's EMEA has laid down clear guidance on in-use stability testing of Human Medicinal Products (CPMP/QWP/2934/99). Additionally, storage stability testing of the sterile solution was performed wherein the solution filled in the multiple dose pen injection device was subjected to stability studies at 25°C/60 % RH for a period of 6 months and at 2-8°C for a period of 12 months and it was observed that the solution stored in the multiple dose pen injection device when kept at 2-8°C or at accelerated stability condition such as 25°C/60 % RH for a period of 6 months remained stable, in terms of both the assay of octreotide as well as assay of phenol. The sterile solution of octreotide acetate can be provided as a reservoir in the multiple dose pen injection device in variable volumes ranging from 10 µL to 40 µL with desired dose dialing facility and appropriate markings for 50, 100, 150, 200 indicating dose in µg, respectively. This designed configuration of the multiple dose injection device of the present invention allows administration of variable doses prescribed for different indications and the dose is based on the severity of the symptoms. For example, the recommended starting dose for treating acromegaly is 50µg administered twice or three times daily. This dose can be self administered using the multiple dose injection device of the present invention. Thus, the multiple dose pen injection device of the present invention confers several advantages. The multiple dose pen injection device of the present invention not only improves accuracy of dose delivery and ease of use for self-injection, but also allows in embodiments, where octreotide acetate is present in more concentrated form such as for instance 5 mg/ml in comparison to Sandostatin® which contains either 200µg/mL or 1mg/mL of octreotide acetate. This is advantageous as lesser volume of solution needs to be injected per dose. For instance, the sterile solution of the present invention having 5mg/ml octreotide acetate advantageously delivers 100µg of octreotide acetate in just 0.02mL of solution instead of 0.5mL or 0.1mL (respectively for 200µg/mL or 1000µg/mL strength) of Sandostatin®. Thus volume per dose is reduced to at least 5 times as compared to the conventional syringe type of product. Further, it was observed that such concentrated solution showed desired osmolarity of 350 mOsm/Kg. This gives added advantage since such subcutaneous injections are associated with less pain at the injection site compared to hyper-osmolar solutions. Besides this, when the capacity of the cartridge of the multiple dose injection device is 3.0 ml, such reservoir of solution can provide multiple portions sufficient to deliver significantly more number of doses. For instance, for treatment of carcinoid tumour, a total daily dose ranging from 100 to 600mcg is prescribed which can be given in 2 to 4 daily divided doses. Thus, for maximum daily dosage of 600 µg, the sterile solution having concentration of 5mg/mL octreotide acetate filled in the pen injection device is sufficient to deliver the said dose for 25 days. As against this, the approved marketed product Sandostatin® available in a 5.0 mL multi-dose vial and has two strengths 200µg/mL and 1000µg/mL; so for a total daily dose of 600 µg the vial will be sufficient for 1.66 days and 8.33 days, respectively.

The sterile solution of the present invention is particularly advantageous as the amount of preservative in the sterile solution of the present invention injected daily, would be reduced, apart from reduction of volume as that of the prior art known octreotide subcutaneous solutions available in the regulated markets. Since the amount of octreotide acetate is higher compared to marketed product Sandostatin®, it was found that a lower amount of preservative per dose as compared to the preservative in marketed product, Sandostatin®, could maintain sterility of the reservoir of the present invention. For instance, Sandostatin® solution present in multidose vial contains 200 µg ocreotide acetate and 5 mg phenol, so in order to deliver recommended dose of 300 µg ocreotide acetate per day (for VIPomas therapy), one needs to inject 1.5 mL of the solution which comprises 7.5 mg of Phenol. While in one embodiment of the present invention wherein octreotide acetate is present at 5mg (5000µg) per ml and phenol is present at 5.0 mg per ml, for administering 300 µg octreotide acetate per day (for VIPomas therapy), one would need to inject 0.06 mL of solution, which includes 0.3 mg phenol, which is significantly lesser than the amount of phenol in marketed product, Sandostatin®. The sterile solution of the present invention thus confers the advantage of reduction in the amount of preservative injected per day (per dose) of drug compared to existing marketed product (Sandostatin®).

According to the invention there is provided a 'multiple dose pen injection device' comprising a reservoir of a sterile solution comprising octreotide acetate and a pharmaceutically acceptable vehicle, the device being adapted to subcutaneously inject a portion of the said reservoir in multiple daily doses and the device further being adapted to provide multiple portions of the said solution, while the reservoir remains sterile. Thus, suitably, the sterile solution is essentially a multi-dose preparation, that is, the sterile reservoir of the solution that can be used for delivering multiple doses. Preferably, a reservoir of the sterile solution is present in the multiple dose pen device and a portion of that reservoir is withdrawn for each administration. The portion suitably contains a desired dose of the octreotide acetate. Suitably, the multiple dose pen injection device according to one embodiment of the present invention comprises a cap covering a cartridge holder, that holds the cartridge filled with the reservoir of sterile, preserved octreotide solution and a dose dialing facility having a dose dial with varied markings visible through a dose window and a dose set knob that allows the user to select the desired dose. In one embodiment, the dose dial can have marking '0' depicting the initial starting position, markings 50; 100; 150; 200 for dosing 50µg 100µg, 150 µg and 200 µg of octreotide acetate, respectively. In one embodiment, the multiple dose pen injector device may include design feature like provision for last dose management such that once the last full dose is delivered the remaining dose cannot be delivered. This additional safety features prevents delivery of insufficient last/remaining dose, thus preventing users from receiving incomplete doses. Further, pen injectors may optionally include a design feature which provides end of injection indication to the user. Also, it is possible to incorporate design feature in the pen injector that can provide end of injection indication to the user such as a visual feature that the dose set knob position & dose markings return to zero/starting position or an audible feature wherein the clicking stops at end of injection. The multiple dose pen injection device thus improves accuracy of dose delivery and ease of use for self-injection. Advantageous properties include portability and ease of reading, ease of dose adjustment, ergonomic design and sturdiness. In one specific embodiment of the present invention, the sterile solution is provided in a multiple dose pen injection device, where the pen injector device consists of a cartridge made up of a USP Type-I Siliconised glass having a specific dimensions viz, total height of 62.30±0.15 mm, body diameter of 11.65±0.15 mm, neck outer diameter of 7.15±0.2 mm, bore diameter of 3.15±0.15 mm and collar thickness of 2.90±0.1mm. Further the pen injector is stoppered with a 10 mm Red Bromobutyl 4023/50 Wester 2223 Sil 4 RFS plunger stopper. Plunger stopper has specific dimensions as outer diameter is 10.0±0.15mm and total height of 8.13±0.3mm. Furthermore, the stoppered glass cartridge is sealed with a Combination RFS Wester Seal of Bromobutyl rubber 4780/40 Cram/Outer 7778/40 Gray having dimensions as, outer diameter of 7.66±0.54mm, inner diameter is 7.5+0.1, -0 and total height of 5.3+0.1, -0.2. The sterile solution is found to be stable in the cartridge having a plunger stopper and Combiseal which is made up of bromobutyl rubber material. The multiple dose pen injection device of the present invention can be used by following the below steps: the user needs to attach a new needle on the tip of the cartridge holder, prime the pen prior to first use, select the dose and inject the sterile solution by pushing the release button. The dose dialing facility enables the priming of multiple dose pen injection device prior to first use, where the dose set knob is set to the appropriate priming unit (200) on the dose dial and the pressing of release button until a stream of sterile solution oozes out from the needle to ensure the removal of any air bubble inside the cartridge. Once the priming is done, the dose to be administered is selected on the dose dial and the sterile solution is injected by pushing the release button. The sterile solution may be prepared by the process whereby specified volume of water for injection was collected in suitable container at a temperature of 20°C to 25°C. To this octreotide acetate was added under stirring to form a clear solution. Specified quantity of lactic acid was dissolved in above solution under stirring until the clear solution was obtained. Specified quantity of mannitol was dissolved in the above solution under stirring until the clear solution was obtained. Separately phenol was dissolved in the specified quantity of water and added to the above bulk solution. Further, the pH of the solution was adjusted to about 4.10± 0.10 with sufficient quantity of sodium bicarbonate solution. This unfiltered bulk solution was stored under nitrogen blanket until before subjecting to membrane filtration. Due to high sensitivity to heat, octreotide solution is sterilized by non heat sterilization processes. In one embodiment, the bulk sterile solution of octreotide acetate is sterilized by membrane filtration through 0.45 micron Nylon 66 capsule filter and 0.2 micron Nylon 66 capsule and then filled into the cartridges. The cartridges are first pre sterilized and then the solutions were filled aseptically under laminar flow. The material of construction of the cartridge may be either glass or plastic. Depending upon the material, a suitable sterilization process can be adapted. However, the sterile solution of the present invention which is intended to deliver multiple doses, is not terminally sterilized.

The following examples illustrate the scope of the present invention without any limitation thereto.

### Example 1-2

The sterile solution of the present invention is prepared according to the formula described in Table 1.

**Table 1: sterile solution of the present invention**

| Sr. No. | Ingredients | Example 1 | | Example 2 | |
|---|---|---|---|---|---|
| | | Concentration in mg/ml | Amount in mg per dose | Concentration in mg/ml | Amount in mg per dose |
| 1 | Octreotide acetate | 5.0 | 0.05 | 5.0 | 0.05 |
| 2 | Lactic acid | 3.4 | 0.034 | 3.4 | 0.034 |
| 3 | Mannitol | 45.0 | 0.45 | 45.0 | 0.45 |
| 4 | Phenol | 4.0 | 0.04 | 5.0 | 0.05 |
| 5 | Sodium bicarbonate | q.s.to pH 4.2±0.3 | | | |
| 6 | Water for injection | q.s to 1 ml | q.s to 0.01 ml | q.s to 1 ml | q.s to 0.01 ml |
| **Pack style** | 3.0 ml multiple use cartridges with multiple dose pen injector device having dose dialing facility | | | | |

Specified volume of water for injection was collected in suitable container at a temperature of 20°C to 25°C. To this octreotide acetate was added under stirring to form a clear solution. Specified quantity of lactic acid was dissolved in above solution under stirring until the clear solution was obtained. Specified quantity of mannitol was dissolved in the above solution under stirring until the clear solution was obtained. Separately phenol was dissolved in the specified quantity of water and added to the above bulk solution. Further, the pH of the solution was adjusted to about 4.10± 0.10 with sufficient quantity of sodium bicarbonate solution. This unfiltered bulk solution was stored under nitrogen blanket until before subjecting to membrane filtration. The bulk solution of octreotide acetate was sterilized by aseptic filtration through a set of 0.45 micron Nylon 66 capsule filter and 0.2 micron Nylon 66 capsule filter and filtered solution was stored under nitrogen blanket. Finally pre-sterilized cartridges were filled aseptically with bulk solution with standard fill volume and stoppered.

Preservative efficacy test (PET) was carried out on the sterile solutions of as per US Pharmacopoeia. It was found that at 5 mg per ml concentration, 4-5 mg per ml of phenol was sufficient to preserve the solution. Further, the osmolality of the sterile solution of octreotide acetate was in the range of 350-390 mOsm/kg, inspite of five times increased concentration of octreotide acetate as compared to the approved product. A concentration of 4-5 mg per ml was sufficient to preserve the solution for the shelf life of the product. This is particularly advantageous as the amount of preservative in the sterile solution of the present invention injected daily, would be reduced, apart from reduction of volume as that of the prior art known octreotide subcutaneous solutions available in the regulated markets.

### Example 3

Storage stability testing - The sterile solution of example 2 contained in the multiple dose pen injector device was subjected to stability studies at 25°C/60 % RH for a period of 6 months and at 2-8°C for a period of 12 months. In this storage period, the device was not used i.e. no solution was withdrawn and the cartridge was not punctured. It was analyzed for the assay and related substances. Assay of phenol was also determined. The results are tabulated in the table 2 below.

**Table 2: Result of stability study of octreotide acetate injection, 5000mcg/ml**

| | Assay | | Related Substances | | | | pH | | Absorba nce at 420 nm | Transmittance at 650 nm |
|---|---|---|---|---|---|---|---|---|---|---|
| | Octreotide | Phenol | Impurity B | Impurity C | Impurity D | Highest Unknown. Impurity | | | | |
| Limits | | | NMT 0.5% | NMT 0.5% | NMT 1.0% | NMT 0.5 % | 3.9-4.5 | | NMT 0.05 | NLT 95 |
| Initial | 98.85 | 106.3 | 0.27 | BQL | 0.09 | 0.23 | 4.1 | | 0 | 100 |
| **25°C/ 60 % RH** | | | | | | | | | | |
| 1M | 101.21 | 106.2 | 0.31 | | BQL | 0.22 | 4.1 | | 0 | 99.9 |
| 2M | 101.45 | 104.8 | 0.20 | | BQL | 0.38 | 4.2 | | 0 | 100 |
| 3M | 99.05 | 98.8 | 0.28 | | 0.1 | 0.48 | 4.1 | | 0 | 100 |
| 6M | 95.23 | 98.1 | 0.35 | | 0.1 | 0.81 | 4.3 | | 0 | 100 |
| **2-8°C** | | | | | | | | | | |
| 3M | 98.38 | 99.1 | 0.27 | | BQL | 0.24 | 4.2 | 0 | | 100 |
| 6M | 95.09 | 98.6 | 0.32 | | BQL | 0.21 | 4.3 | 0 | | 100 |
| 12 M | 96.92 | 103.8 | 0.33 | | ND | 0.182 | 4.2 | 0 | | 100 |

The results of the aforesaid stability tests indicated that the solution stored in the multiple dose pen injection device when kept at 2-8°C for a period of 12 months or at 25°C/60 % RH for a period of 6 months, remains stable, in terms of both the assay of octreotide acetate as well as assay of phenol preservative, and the related substance/impurities remained within limits, thus indicating that the sterile solution of the present invention in the multiple dose pen injector device remains stable on storage throughout the shelf life of the product.

### Example 4

The sterile solutions of Example 1 and Example 2 were subjected to antimicrobial effectiveness testing to evaluate the efficacy of the preservative, phenol. The test is carried out as per United States Pharmacopoeia USP33 NF28. The solutions were stored at accelerated stability conditions and then subjected to the antimicrobial testing. The results of the test are given below:

**Table 3: Preservative efficacy test of octreotide acetate Injection 5000 mcg/ml**

| Example | Observation | | |
|---|---|---|---|
| | Initial | 25°C/60% RH | |
| | | 3 Months | 6 Months |
| 2 | Complies | Complies | Complies |

It was found that the an approved amount of phenol i.e. 0.5 % or 0.4 % (80 % of the approved quantity) was sufficient to keep the sterile solution of the present invention having five times higher concentration of octreotide acetate, in a sterile condition. The solutions at initial as well as at accelerated stability conditions at phenol concentrations of 0.5 % and 0.4 % were complying with USP criteria.

## Claims

1. A multiple dose pen injection device comprising a reservoir of a sterile solution comprising from 2 mg to 20 mg of octreotide acetate per milliliter of the solution and at least one preservative in a pharmaceutically acceptable vehicle, the device being adapted to subcutaneously inject a portion of the said reservoir in multiple daily doses and further being adapted to provide multiple portions of the said solution, while the reservoir remains sterile.

2. The multiple dose pen injection device as claimed in claim 1 wherein the preservative is selected from phenol, benzyl alcohol, m-cresol, methyl parabens, propylparaben, butylparaben, chlorbutanol, thimerosal, and phenylmercuric salts.

3. The multiple dose pen injection device as claimed in claim 2 wherein the preservative is phenol and is present in an amount from 4 mg to 5 mg per milliliter of the solution.

4. The multiple dose pen injection device as claimed in any preceding claim wherein octreotide acetate is present in amount of 5 mg per milliliter of the solution.

5. The multiple dose pen injection device as claimed in claim 4 wherein the solution present as reservoir provides octreotide acetate for 25 days when administered at a total daily dose of 600 µgs.

6. The multiple dose pen injection device as claimed in claim 4 wherein the solution present as reservoir provides octreotide acetate for 50 days when administered at a total daily dose of 300 µgs.

## Patentansprüche

1. Mehrfachdosis-Peninjektionsvorrichtung, umfassend ein Reservoir einer sterilen Lösung, umfassend 2 mg bis 20 mg Octreotidacetat pro Milliliter der Lösung und mindestens einen Konservierungsstoff in einem pharmazeutisch unbedenklichen Vehikel, wobei die Vorrichtung angepasst ist, um subkutan einen Teil des Reservoirs in mehreren Tagesdosen zu injizieren und ferner angepasst ist, um mehrere Teile der Lösung bereitzustellen, während das Reservoir steril bleibt.

2. Mehrfachdosis-Peninjektionsvorrichtung nach Anspruch 1, wobei der Konservierungsstoff ausgewählt ist aus Phenol, Benzylalkohol, m-Cresol, Methylparabenen, Propylparaben, Butylparaben, Chlorbutanol, Thimerosal und Phenylquecksilbersalzen.

3. Mehrfachdosis-Peninjektionsvorrichtung nach Anspruch 2, wobei der Konservierungsstoff Phenol ist und in einer Menge von 4 mg bis 5 mg pro Milliliter der Lösung vorliegt.

4. Mehrfachdosis-Peninjektionsvorrichtung nach einem der vorhergehenden Ansprüche, wobei Octreotidacetat in einer Menge von 5 mg pro Milliliter der Lösung vorliegt.

5. Mehrfachdosis-Peninjektionsvorrichtung nach Anspruch 4, wobei die Lösung, die als Reservoir vorliegt, Octreotidacetat 25 Tage lang bereitstellt, wenn es mit einer Tagesdosis von 600 µg verabreicht wird.

6. Mehrfachdosis-Peninjektionsvorrichtung nach Anspruch 4, wobei die Lösung, die als Reservoir vorliegt, Octreotidacetat 50 Tage lang bereitstellt, wenn es mit einer Tagesdosis von 300 µg verabreicht wird.

## Revendications

1. Dispositif d'injection à stylo à doses multiples comprenant une réserve de solution stérile comprenant de 2 mg à 20 mg d'acétate d'octréotide par millilitre de solution et au moins un conservateur dans un véhicule pharmaceutiquement acceptable, le dispositif étant adapté pour injecter par voie sous-cutanée une portion de ladite réserve en de multiples doses quotidiennes et étant en outre adapté pour fournir de multiples portions de ladite solution, alors que la réserve reste stérile.

2. Dispositif d'injection à stylo à doses multiples selon la revendication 1, ledit conservateur étant choisi parmi le phénol, l'alcool benzylique, le m-crésol, les méthylparabènes, le propylparabène, le butylparabène, le chlorobutanol, le thimérosal et les sels phénylmercuriques.

3. Dispositif d'injection à stylo à doses multiples selon la revendication 2, ledit conservateur étant le phénol et étant présent en une quantité de 4 mg à 5 mg par millilitre de solution.

4. Dispositif d'injection à stylo à doses multiples selon l'une quelconque des revendications précédentes, ledit acétate d'octréotide étant présent en une quantité de 5 mg par millilitre de solution.

5. Dispositif d'injection à stylo à doses multiples selon la revendication 4, ladite solution présente sous forme de réserve fournissant de l'acétate d'octréotide pendant 25 jours lorsqu'il est administré à une dose quotidienne totale de 600 µg.

6. Dispositif d'injection à stylo à doses multiples selon la revendication 4, ladite solution présente sous forme de réserve fournissant de l'acétate d'octréotide pendant 50 jours lorsqu'il est administré à une dose quotidienne totale de 300 µg.
